(19) Europäisches Patentamt
European Patent Office
Office européen des brevets

(11) **EP 1 974 759 A1**

(12) **EUROPÄISCHE PATENTANMELDUNG**

(43) Veröffentlichungstag:
**01.10.2008 Patentblatt 2008/40**

(51) Int Cl.:
*A61M 5/315* (2006.01)

(21) Anmeldenummer: **07108190.5**

(22) Anmeldetag: **14.05.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(30) Priorität: **29.03.2007 EP 07105279**

(71) Anmelder:
• **Schmidt-Evers, Jürgen**
  **82211 Herrsching (DE)**
• **Ruhland, Bernd**
  **81377 München (DE)**

• **Micheler, Clemens**
  **82319 Starnberg (DE)**

(72) Erfinder:
• **Schmidt-Evers, Jürgen**
  **82211 Herrsching (DE)**
• **Ruhland, Bernd**
  **81377 München (DE)**
• **Micheler, Clemens**
  **82319 Starnberg (DE)**

(74) Vertreter: **Schmidt-Evers, Jürgen**
  **Rehmstrasse 15**
  **82211 Herrsching (DE)**

(54) **Injektionsgerät, insbesondere Insulin-Pen**

(57)    Um herkömmliche Insulin-Pens kürzer machen zu können, wird vorgeschlagen, entweder den Kolbenstößel (8) aus mindestens drei teleskopisch aus- und einfahrbaren Stößelteilabschnitten (8a, 8b, 8c) zusammenzusetzen oder den Kolbenstößel (28) durch das Kolbenelement (4) hindurchzuführen und gegenüber dem Kolbenelement (4) verschiebbar zu machen.

Fig. 4

EP 1 974 759 A1

Fig. 9

**Beschreibung**

Die Erfindung betrifft ein Injektionsgerät, insbesondere einen Insulin-Pen gemäß dem Oberbegriff der Ansprüche 1 und 7.

Insulin-Pens der vorstehend beschriebenen Art sind millionenfach in Gebrauch. Es gibt weltweit derzeit etwa zehn Hersteller für derartige Insulin-Pens. Die einzelnen Typen haben sich weitgehend angenähert. Die Pens haben die Form eines übergroßen und überdicken Füllfederhalters mit einer Länge von etwa 15 cm ohne Injektionskanüle oder 17 cm mit einer solchen. Ihr Gehäuse besteht aus mindestens zwei Gehäuseteilen. Der erste Gehäuseteil dient zur Aufnahme einer den Zylinderraum enthaltenden Insulin-Patrone (später auch "Behälter" bezeichnet), der zweite Gehäuseteil enthält einen Stößelmechanismus sowie eine Dosiervorrichtung. Die Insulin-Patronen bestehen aus transparentem Material, vorzugsweise Glas. Der erste Gehäuseteil weist meistens Fensteröffnungen auf, durch die der Füllstand der Insulin-Patronen sichtbar ist.

Der Zylinderraum der Insulin- Patronen ist an einem Ende mit einem pfropfenartigen elastischen Kolbenelement verschlossen. Das andere Ende der Insulin-Patrone ist verjüngt und ebenfalls verschlossen, und zwar mit einer Membran, der aber von der Injektionskanüle leicht durchstochen werden kann, so dass die Injektionskanüle dann Verbindung zu dem Zylinderraum hat. In dem Zylinderraum befindet sich das flüssige Insulin. Wenn auf das Kolbenelement in Längsrichtung der Insulin-Patrone Druck ausgeübt wird, so verschiebt sich in ihr das Kolbenelement, mit der Folge, dass das Injektionsmittel unter Druck über die Injektionskanüle austritt.

Der im zweiten Gehäuseteil befindliche Stößelmechanismus enthält einen Kolbenstößel, der auf das Kolbenelement verschiebend einwirkt. Bei fast allen bisher bekannten Insulin-Pens ist der Kolbenstößel steif und hat eine Länge, die mindestens gleich dem maximalen Gesamtverschiebeweg des Kolbenelementes in der Insulinpatrone zwischen deren Füll- und Leerzustand oder größer als dieser Gesamtverschiebeweg ist. Wenn eine neue Insulin-Patrone zur Anwendung kommt, muss der Kolbenstößel vollständig in das zweite Gehäuseteil zurückgezogen werden. Die theoretische Mindestlänge der hier betrachteten Insulin-Pens ist deshalb im wesentlichen bestimmt durch die Länge der Insulin-Patrone zuzüglich der Länge des Kolbenstößels. Die Insulin-Patronen haben eine Länge von circa 6,5 cm, und der maximale Verschiebeweg des Kolbenelementes in der Insulin-Patrone beträgt etwa 4,5 cm. Das bedeutet, dass die Gesamtlänge eines Insulin-Pens der vorstehend beschriebenen Konstruktion nicht unter 11 cm (ohne Injektionskanüle) liegen kann. Da die Dosiervorrichtung in dem zweiten Gehäuseteil außerdem noch Platz braucht, kommt man zu der oben angegebenen Gesamtlänge von circa 15 cm (ohne Injektionskanüle).

Insulin-Pens der hier betrachteten Art sind etwas mehr als zwanzig Jahre auf dem Markt. Es gibt eine große Zahl von Schutzrechtsveröffentlichungen, von denen nachfolgend einige genannt werden sollen:

EP 0 245 312 B1, EP 0 450 905 B1, EP 0 496 141 B1, EP 0 829 268 A2, EP 1 644 061 B1, DE 37 16 340 C2, DE 197 30 999 C1, US 4,865,591 und WO 99/38554.

In letzter Zeit ist jedoch bei den Benutzern der Insulin-Pens der Wunsch nach kürzeren Insulin-Pens entstanden, die möglichst auch ein modernes und ansprechendes Design haben sollen. Dieser Wunsch entspringt der Tatsache, dass ein Diabetiker seinen Insulin-Pen ständig bei sich tragen muss, um sich bis zu dreimal oder mehr am Tag selbst die notwendige Menge Insulin zu injizieren. Dabei ist es unvermeidlich, dass der Insulin-Pen auch von dritten Personen bemerkt wird. Es liegt auf der Hand, dass ein solches Gerät sowohl bei dem Benutzer als auch bei einem neutralen Betrachter umso mehr Akzeptanz findet, je kleiner bzw. kürzer dieses ist. Eine kürzere Bauweise eröffnet zudem die Möglichkeit, das bisherige nahezu einheitliche Design zu verlassen und dem Gerät ein neues und modernes Design zu geben.

Dem vorstehend beschriebenen Wunsch der Benutzer von Insulin-Pens wurde bereits von einem Gerätehersteller Rechung getragen. Dieser hat ein Insulin-Injektionsgerät mit entwickelt, bei dem sich der Stößelmechanismus seitlich neben der Insulin-Patrone befindet. Der Kolbenstößel selbst ist flexibel und nach Art eines Bowden-Zuges um den Rand der Insulin-Patrone herumgeführt, sodass er in deren Inneres eintauchen und das Kolbenelement verschieben kann. Dieses Gerät ist Gegenstand der folgenden Schutzrechtsveröffentlichung: EP 0 110 687 B1, EP 0 721 358 B1, EP 0 909 194 B1.

Das vorstehend genannte Gerät hat eine Länge von circa 13cm (ohne Injektionskanüle). Damit der Stößelmechanismus jedoch seitlich neben der Insulin-Patrone Platz finden kann, hat das Gerät in einer Querschnittsrichtung eine Abmessung von ca. 4 cm, wohingegen die herkömmlichen oben beschriebenen Insulin-Pens einen Zylinderdurchmesser von etwa 2 cm haben.

Der Erfindung liegt die Aufgabe zugrunde, ein Injektionsgerät, insbesondere einen Insulin-Pen zu schaffen, der ohne wesentliche Vergrößerung der Dicke in beachtlichem Maße kürzer als die eingangs beschriebenen herkömmlichen Insulin-Pens ist.

Ein erster Lösungsvorschlag für diese Aufgabe ist gemäß Anspruches 1 dadurch gekennzeichnet, dass der Kolbenstößel aus mindestens drei teleskopisch aus- und einfahrbaren Stößelteilabschnitten besteht, von denen jeder im eingefahrenen Zustand kürzer als der maximale Gesamtverschiebungsweg des Kolbenelementes in dem Behälter zwischen dem Füll- und dem Leerzustand des Behälters ist.

Die Insulin-Pens nach dem Stand der Technik mit steifem Kolbenstößel haben zwar bereits zwei konzentrische Stößelabschnitte; beide sind jedoch mindestens so lang wie der Gesamtverschiebeweg des Kolbenelementes. Durch die Verwendung von drei oder mehr Stößelabschnitten, die konzentrisch ineinander angeordnet sind und etwa die gleiche Länge haben, ist eine beachtliche Verkürzung des Insulin-Pens möglich.

Für drei Stößelabschnitte ergibt sich die theoretische Verkürzung (ohne Berücksichtigung einer Überlappung im ausgefahrenen Zustand) wie folgt:

Es sei vereinfachend vorausgesetzt, dass die beiden Gehäuseteile bei zwei konzentrischen Stößelabschnitten (Stand der Technik) die gleiche (durch die Insulin-Ampulle bestimmte) Länge A haben, so dass bei ausgefahrenen Stößelabschnitten jeder Stößelabschnitt sich durch einen der beiden Gehäuseteile erstreckt. Die Gesamtlänge beider Gehäuseteile ist dann

$$L_1 = 2A$$

Bei drei Stößelabschnitten muss das erste Gehäuseteil wiederum die (durch die Insulin-Ampulle bestimmte) Länge A haben, während die Länge des zweiten Gehäuseteiles mit X angesetzt werden soll. Die Gesamtlänge des Gehäuses ist dann

$$L_2 = A + X$$

Die drei Stößelabschnitte sollen alle gleich lang sein, so dass sie im zusammengefahrenen Zustand die geringste mögliche Länge haben und in diesem Zustand gerade noch in dem zweiten Gehäuseteil Platz finden. Das bedeutet, dass sie im zusammengefahrenen Zustand und einzeln die Länge X haben. Im ausgefahrenen Zustand haben sie dann die Gesamtlänge des Gehäuses, also

$$L_2 = 3X$$

Daraus ergibt sich für X = 1/2A und für die Gesamtlänge des Gehäuses

$$L_2 = 3/2 \ A$$

Die erzielte Längenreduzierung ist dann

$$L_2/L_1 = 3/4A$$

Das ist gleichbedeutend mit einer Reduzierung der Gesamtlänge um 25%. Bei vier Stößelabschnitten erreicht man eine theoretische Reduzierung der Gesamtlänge um 33%.

Weiterbildungen des ersten Lösungsvorschlags sind Gegenstand der Ansprüche 1-6.

Ein zweiter Lösungsvorschlag für die oben angegebene Aufgabe besteht darin, dass der Kolbenstößel das Kolbenelement durchgreift und in den das Injektionsmittel enthaltenen Teil des Zylinderraumes hineintaucht, und dass das Kolbenelement relativ gegenüber dem Kolbenelement verschiebbar ist.

Der zweite Lösungsvorschlag erlaubt theoretisch eine Verkürzung des Insulin-Pens um nahezu 50%. Wenn noch eine Dosiervorrichtung vorgesehen werden soll, ist bei diesem Lösungsvorschlage eine Längenreduzierung um ca. 44% realistisch.

Eine Weiterbildung des zweiten Lösungsvorschlages ist Gegenstand des Anspruches 8.

Da der Kolbenstößel zur Vorbereitung eines Injektionshubes ein kleines Stück gegenüber dem Kolbenelement zurückgezogen werden muss, hinterlässt der Kolbenstößel ein entsprechendes negatives Tauchvolumen, das dazu führen würde, dass der Insulin-Pen über die Injektionskanüle Luft ansaugt. Das ist jedoch unerwünscht, weil das Insulin vor der Injektion möglichst nicht mit Luft in Verbindung kommen sollte. Um diesem Phänomen entgegenzuwirken, wird vorgeschlagen, Ausgleichsmittel vorzusehen, die Gegenstand der Ansprüche 9-18 sind.

Ausführungsbeispiele der Erfindung werden nachfolgend anhand der Zeichnungen beschrieben. Es zeigen:

Figur 1     eine schematische Darstellung eines Insulin-Pens nach dem Stand der Technik mit den beiden Endpositionen des Kolbenelementes;

Figur 2     eine schematische Darstellung eines Insulin-Pens gemäß dem ersten Erfindungsvorschlag mit den beiden Endpositionen des Kolbenelementes;

Figur 3     eine schematische Darstellung eines Insulin-Pens gemäß dem zweiten Erfindungsvorschlag mit den beiden Endpositionen des Kolbenelementes;

Figur 4     eine etwas konkretisierte, aber immer noch schematisierte Darstellung des ersten Erfindungsvorschlages;

Figur 5     eine etwas konkretisierte, aber immer noch schematisierte Darstellung des zweiten Erfindungsvorschlages;

Figur 6     einen Schnitt VI-VI durch den Insulin-Pen nach Figur 5 mit einer ersten Ausführungsform der Ausgleichsmittel und eines dazu gehörenden Getriebemechanismus;

Figur 7     ein Schnitt, wie Figur 6, jedoch mit der ersten Ausführungsform der Ausgleichsmittel, jedoch einem alternativen Getriebemechanismus;

Figur 8     fünf verschiedene Phasen der Injektion mit einem Insulin-Pen gemäß dem zweiten Erfindungsvorschlag mit der ersten Ausführungsform der Ausgleichsmittel nach Figur 7;

Figur 9     eine etwas konkretisierte, aber immer noch schematisierte Darstellung des zweiten Erfindungsvorschlages mit der ersten Ausführungsform der Ausgleichsmittel, jedoch einem weiteren alternativen Getriebemechanismus;

Figur 10    einen Schnitt XI - XI durch Figur 9;

Figur 11    einen Schnitt XII - XII durch Figur 9;

Figur 12    einen Schnitt XIII - XIII durch Figur 9;

Figur 13    eine schematisierte Teildarstellung einer zweiten Ausführungsform der Ausgleichsmittel.

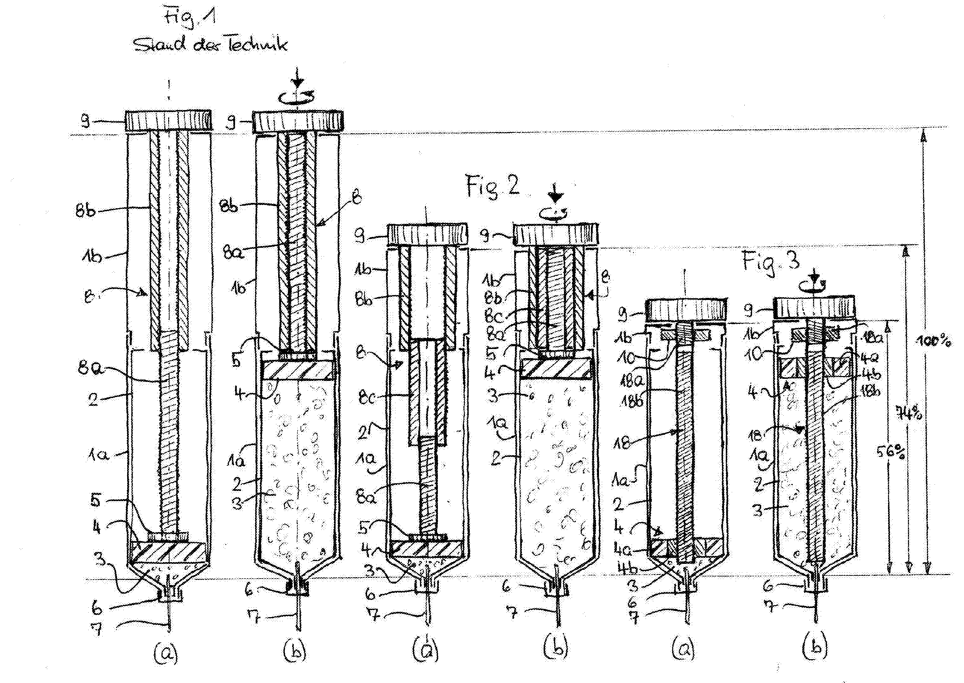

In den Figuren 1(a) und (b) ist ein Insulin-Pen nach dem Stand der Technik in stark schematisierter Form dargestellt. Der Insulin-Pen weist einen ersten Gehäuseteil 1a und einen zweiten Gehäuseteil 1b auf. Die beiden Gehäuseteile 1a und 1b sind in der Regel durch eine Schraubverbindung miteinander verbunden. In dem ersten Gehäuseteil 1a befindet sich eine aus Glas bestehende zylindrische Insulin-Patrone 2, die nachfolgend als "Behälter" bezeichnet wird und das flüssige Insulin 3 enthält. Der Zylinderraum ist oben mit einem verschiebbaren Kolbenelement 4 verschlossen. Auf das untere Ende des ersten Gehäuseteiles 1a ist eine Abschlusskappe 6 aufgeschraubt, die eine Injektionskanüle 7 enthält. Die Injektionskanüle 7 durchgreift einen Gummiverschluss am unteren Ende des Behälters 2 und steht mit dem Zylinderraum in Verbindung. Die Insulin-Patrone 2 muss keinen kreisrunden Querschnitt haben, es sind auch andere Querschnittsformen möglich.

Der zweite Gehäuseteil 1b enthält eine Dosiervorrichtung und einen Stößelmechanismus 8. Die Dosiervorrichtung ist in der Regel eine relativ komplizierte Mechanik, die - da sie nicht Gegenstand der Erfindung ist - aus Gründen der Vereinfachung und Schematisierung als Drehknopf 9 dargestellt ist, mit dem der Injektionshub einstellbar ist. Der ebenfalls vereinfacht dargestellte Stößelmechanismus 8 besteht aus einer fest mit dem Drehknopf 9 verbundenen Hülse 8b mit Innengewinde, die sich in dem zweiten Gehäuseteil 1b befindet und sich auch beim Drehen des Drehknopfes 9 nicht aus diesem herausbewegt. In der Gewindehülse 8b befindet sich ein Kolbenstößel 8a mit Außengewinde. Der Kolbenstößel 8a sitzt mit einem tellerartigen Verbindungselement 5 auf dem Kolbenelement 4 auf und ist mit dem Verbindungselement 5 fest verbunden. Das tellerartige Verbindungselement 5 hat mit dem Kolbenelement 4 in Reibschluss.

Figur 1(b) zeigt das Kolbenelement 4 in der Ausgangsposition zu Beginn der Injektionen, während Figur 1(a) das Kolbenelement in der Endposition, also am Ende des Gesamtverschiebeweges in dem Behälter 2 zeigt, nachdem nahezu das gesamte Insulin 5 verbraucht worden ist.

Beim Drehen des Drehknopfes 9 in der durch den Pfeil oberhalb des Drehknopfes in Figur 1(b) angedeuteten Richtung, schraubt sich die Gewindehülse 8b mit dem Drehknopf nach oben, während sich der Kolbenstößel 8a mit dem tellerartigen Verbindungselement 5 auf dem Kolbenelement 4 abstützt und nicht mit der Gewindehülse mitgedreht wird. Nachdem sich auf diese Weise der Drehknopf 9 in Figur 1(b) etwas über den zweiten Gehäuseteil erhoben hat, kann durch Druck auf den Drehknopf von oben, wie dies durch den vertikalen Pfeil angedeutet ist, ein Injektionshub ausgeführt werden. Der Hub wird durch die Drehung des Drehknopfes eingestellt und vorbereitet. Der Drehknopf 9 wirkt also hier wie eine Dosiervorrichtung.

Durch mehrmaliges Einstellen des Injektionshubes in der vorstehend beschriebenen Weise und durch das Ausüben eines Injektionshubes ebenfalls in der vorstehend beschriebenen Weise gelangt das Kolbenelement 4 schließlich in die in Figur 1(a) gezeigte Endposition.

Aus Figur 1 kann man entnehmen, dass der Kolbenstößel 18 mindestens gleich lang wie oder länger als der Gesamtverschiebeweg des Kolbenelements 4 in dem Behälter 2 sein muss.

Der in Figur 2 gezeigte Insulin-Pen gemäß dem ersten Erfindungsvorschlag unterscheidet sich von dem in Figur 1 gezeigten Insulin-Pen nach dem Stand der Technik dadurch, dass der Kolbenstößel 8 hier aus drei teleskopisch aus- und einfahrbaren Stößelteilabschnitten 8a, 8b und 8c besteht. Die drei Stößelabschnitte sind konzentrisch ineinander angeordnet. Der innerste Stößelabschnitt 8c hat Außengewinde und sitzt wiederum mit einem tellerartigen Verbindungselement auf dem Kolbenelement 4 auf. Der mittlere Stößelabschnitt 8c hat Innen- und Außengewinde. Der äußere Stößelabschnitt 8b hat Innengewinde. Die Gewinde aller drei Stößelabschnitte 8a, 8b, 8c sind gleichsinnig und haben die gleiche Steigung. Der Anfangszustand zu Beginn der Injektionen ist wiederum in Figur 2(b) gezeigt. Hier ist der Behälter 2 noch mit Insulin 3 gefüllt. Die drei Stößelabschnitte 8a, 8b und 8c sind ineinander gefahren.

In Figur 2(a) ist die Endposition gezeigt, in der sich nahezu kein Insulin 3 mehr in dem Behälter befindet. Hier schließen die drei Stößelabschnitte 8a, 8b und 8c bis auf geringfügige Überlappungen aneinander an. Um von dem gefüllten Zustand in Figur 2(b) zu dem entleerten Zustand in Figur 2(a) zu kommen, muss der Drehknopf 9 - wie vorher in Zusammenhang mit Figur 1 beschrieben wurde - gedreht und gedrückt werden. Der äußerste Stößelabschnitt 8b ist mit dem Drehknopf 9 drehfest verbunden. Der innerste Stößelabschnitt 8a ist mit dem tellerartigen Verbindungselement 5 drehfest verbunden und hat Reibschluss mit dem Kolbenelement 4. Das tellerartige Verbindungselement ist dazu unten aufgeraut oder mit einer Zahnung versehen. Durch Drehen des Drehknopfes 9 in der durch den gekrümmten Pfeil angegebenen Richtung dreht sich zunächst der äußerste Stößelabschnitt 8b mit dem Drehknopf 9 nach oben. Wenn nach einer Reihe von Injektionshüben der äußerste Stößelabschnitt 8b seine am weitesten ausgefahrene Position erreicht hat (in dieser wird er gegenüber dem mittleren Stößelabschnitt durch eine nicht dargestellte Arretiervorrichtung, die eine notwendige Überlappung mit dem mittleren Stößelabschnitt 8c gewährleistet, arretiert), so dreht sich als nächstes der mittlere Stößelabschnitt 8c mit dem äußersten Stößelabschnitt 8b gegenüber dem inneren Stößelabschnitt 8a nach oben. Nach weiteren Injektionshüben wird schließlich die in Figur 2(a) gezeigte am weitesten ausgefahrene Stellung erreicht. Dabei wird auch hier die notwendige Überlappung zwischen dem mittleren Stößelabschnitt 8c und dem innersten Stößelabschnitt 8a durch eine nicht dargestellte Arretiervorrichtung gewährleistet. Unterstellt man eine notwendige Überlappung von beispielsweise 5% der Länge der etwa gleich langen Stößelabschnitte 8a, 8b und 8c, so erreicht der aus den drei Abschnitten bestehende Kolbenstößel 8 im ausgefahrenen Zustand eine Länge, die 2,90 mal der Länge eines einzelnen Stößelabschnittes ist. Das bedeutet umgekehrt, dass der zweite Gehäuseteil 1b des Insulin-Pens nach Figur 2 nur etwa ein Drittel der Länge des zweiten Gehäuseteils 1b des bekannten Insulin-Pens nach Figur 1 haben muss. Nimmt man die Gesamtlänge der beiden Gehäuseteile 1a und 1b des bekannten Insulin-Pens nach Figur 1 mit 100% an, so kann mit der erfindungsgemäßen Konstruktion nach Figur 2 eine Längenreduktion um ca. 26% erreicht werden.

Der zweite Erfindungsvorschlag nach Figur 3 unterscheidet sich von den Konstruktionen nach den Figuren 1 und 2 im wesentlichen dadurch, dass der Kolbenstößel 18 nicht mehr nur außen auf dem Kolbenelement 4 aufsitzt, sondern durch dieses hindurchgeführt ist und sich über seine nahezu gesamte Länge innerhalb des Behälters 2 erstreckt. Dabei zeigt wiederum Figur 3(b) die Position des Kolbenelementes 4 zu Beginn einer Injektionskette, also dann, wenn der Behälter 2 noch vollständig mit Insulin 3 gefüllt ist, wohingegen Figur 3(a) die Position des Kolbenelementes 4 zeigt, wenn nahezu kein Insulin 3 mehr in dem Behälter 2 ist. Wesentlich ist, dass mit der Konstruktion nach Figur 3 eine noch stärkere Längenreduktion gegenüber der Konstruktion nach der Figur 1 erreichbar ist, und zwar um etwa 44%.

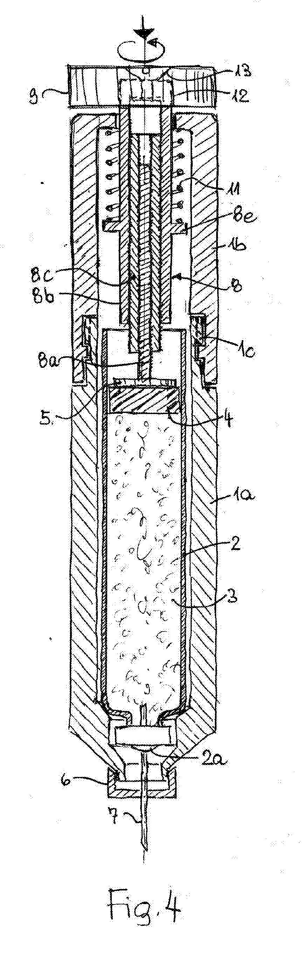

In Figur 4 ist die Konstruktion nach Figur 2, also ein Insulin-Pen gemäß dem ersten Erfindungsvorschlag, allerdings etwas detaillierter; es handelt sich jedoch noch immer um eine schematisierte Darstellung. Der erste Gehäuseteil 1a und der zweite Gehäuseteil 1b sind hier mit einem Bajonettverschluss 1c verbunden. Der äußere Stößelteilabschnitt 8b ist an seinem oberen Ende in einer kreisrunden Ausnehmung des Drehknopfes 9 versenkt und mit einer Schraube 13 festgehalten. Durch diese Verbindung ist es möglich, die einzelnen Teile problemlos zu montieren.

Der äußere Stößelteilabschnitt 8b weist etwa im mittleren Bereich einen tellerartigen Flansch 8e auf, auf dem sich eine Druckfeder 11 mit einer Seite abstützt. Die andere Seite der Druckfeder stützt sich innen an dem zweiten Gehäuseteil 1b ab. Auf diese Weise wird gewährleistet, dass das tellerartige Verbindungselement 5 immer Reibschluss mit dem Kolbenelement 4 hat, und zwar auch dann, wenn der Drehknopf 9 - nachdem er herausgedreht worden ist - nochmals gedreht werden sollte, aber versehentlich in die falsche Richtung.

Ansonsten ist in Figur 4 alles so, wie es in Verbindung mit Figur 2 beschrieben wurde.

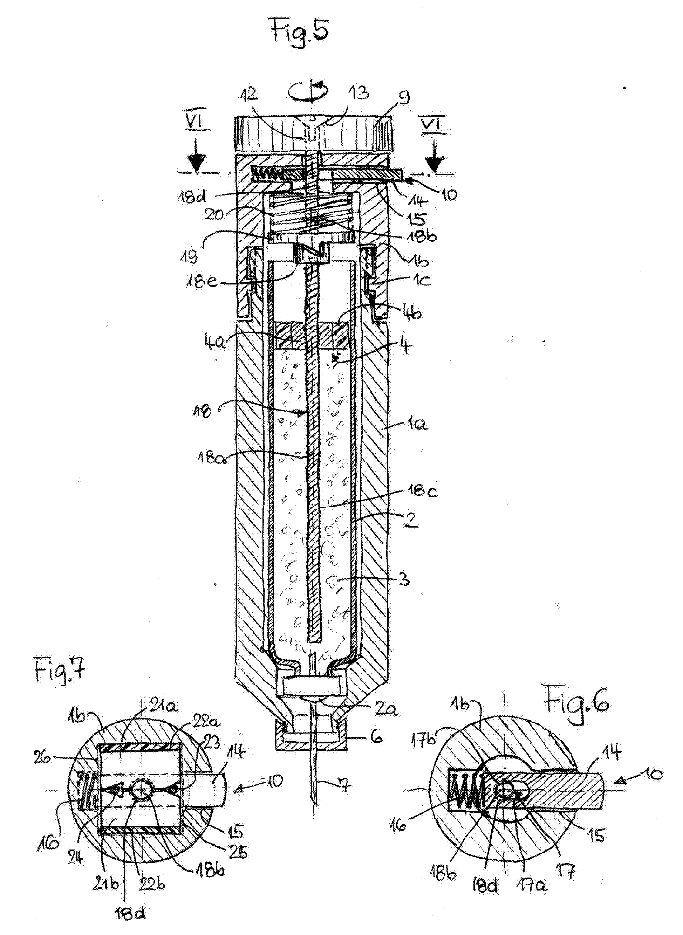

Die Ausführungsform nach Figur 5 zeigt eine etwas konkretere Darstellung des in Figur 3 gezeigten Insulin-Pens. Der Kolbenstößel 18 besteht hier aus einem unteren (ersten) Kolbenstößel-Abschnitten 18a und einem oberen (zweiten9 Kobenstößel-Abschnitt 18b, die über eine nur in einer Drehrichtung wirkende Drehkupplung 18c miteinander verbunden sind. Damit der Kupplungseingriff erhalten bleibt, ist auf dem oberen Kolbenstößel-Abschnitt 18b ein Stützteller 19 angebracht, an dem sich ein Ende einer Druckfeder 20 abstützt, deren anderes Ende sich an einem Vorsprung innerhalb des zweiten Gehäuseteiles 1b abstützt. Die Teilung des Kolbenstößels 18 in einen unteren Kolbenstößel-Abschnitt 18a und einen oberen Kolbenstößel-Abschnitt 18b wurde vorgesehen, um die Montage zu vereinfachen. Wenn die Bajonettverbindung 1c zwischen den beiden Gehäuseteilen 1a und 1b gelöst wird, so können die beiden Gehäuseteile mit den darin befindlichen Elementen problemlos voneinander getrennt werden und umgekehrt. Vorteilhaft ist dabei ferner, dass die Behälter 2 bzw. Insulin-Patronen vom Insulinhersteller nur mit dem unteren Abschnitt 18a des Kolbenstößels 18 versehen und geliefert werden müssen.

Die Drehkupplung 18e zwischen den beiden Kolbenstößel-Abschnitten 18a und 18b ist so gestaltet, dass eine Drehmitnahme immer dann erfolgt, wenn der Drehknopf 9 in Richtung des gekrümmten Pfeils gedreht wird. In dieser Drehrichtung wird der Kolbenstößel 18 aus dem Behälter 2 zur Vorbereitung des Hubes herausgedreht. Wird der Drehknopf 9 andersherum gedreht, so erfolgt keine Drehmitnahme, sondern die Drehkupplung 18e wirkt als Ratsche. Dies ist notwendig, um zu verhindern, dass das Kolbenelement 4 bei einer Drehung des Drehknopfes 9 entgegen der durch den gekrümmten Pfeil angedeuteten Richtung - wenn der Kolbenstößel 18 in dieser Richtung mitgedreht werden würde - von dem Kolbenstößel nach oben gezogen wird. Das hätte nämlich zur Folge, dass über die Injektionskanüle 9 Luft in das Insulin 3 gelangt, was möglichst zu vermeiden ist.

Zu ergänzen ist noch, dass der obere Kolbenstößel-Abschnitt 18b auch hier wieder aus Montagegründen in eine Ausnehmung 12 im Drehknopf 9 eingreift und mittels einer Schraube 13 dort befestigt ist.

Das Kolbenelement 4 enthält im vorliegenden Fall eine Gewindebohrung, die passend zu dem Gewinde 18c des unteren Kolbenstößel-Abschnittes 18a ist. Um sicherzugehen, dass die Kolbenstange 18 in dem Kolbenelement 4 beim Drehen nicht durchrutscht, sondern das Kolbenelement 4 in Längsrichtung transportiert, besteht das Kolbenelement 4 aus einem inneren ringförmigen Kolbenabschnitt 4a und einem äußeren ringförmigen Kolbenabschnitt 4b. Der innere ringförmige Kolbenabschnitt 4a ist aus steiferem Material, welches die Erfüllung der vorstehenden Forderung gewährleistet. Der äußere ringförmige Kolbenabschnitt 4b ist aus elastischem Material, wie Gummi, welches sich eng an die Wand des Behälters 2 anschmiegt und verhindert, dass unter Druck durch den Spalt Insulin hindurch treten kann. Damit auch durch den Gewindespalt zwischen dem unteren Kolbenstößel-Abschnitt 18a und dem inneren ringförmigen Abschnitt 4b des Kolbenelementes 4 kein Insulin unter Druck hindurch treten kann, kann gegebenenfalls in dem Gewindeloch eine Füllung aus hochviskosem Dichtungsmaterial vorgesehen werden. Da das Kolbenelement 4 - wie nachstehend noch beschrieben wird - nur nach unten bewegt werden kann, ist ausgeschlossen, dass das hochviskose Dichtungsmittel in das Insulin befördert wird.

Bei den Konstruktionen gemäß den Figuren 3/5-8 tritt noch ein Phänomen auf, das ggf. einer Korrektur bedarf. Wenn der Kolbenstößel 18 zur Vorbereitung eines Injektionshubes nach oben geschraubt wird, so gibt er einen Teil seines Tauchvolumens in dem Insulin 3 frei, was gleichbedeutend mit der Entstehung eines Vakuums ist. Die Folge wäre, dass durch die Injektionskanüle 7 in unerwünschter Weise Luft eingesaugt wird. Um dies zu verhindern sind Ausgleichsmittel vorgesehen, die mit einem Getriebemechanismus 10 das negative Tauchvolumen des Kolbenstößels 18 kompensieren.

Eine erste Ausführungsform des Getriebemechanismus ist in den Figuren 5 und 6 gezeigt. Hier führt das Kolbenelement 4 eine geringfügige Gegenbewegung aus, damit das negative Tauchvolumen wieder ausgeglichen wird. Das wird dadurch erreicht, dass auch der obere Kolbenstößel-Abschnitt 18b mit einem Gewinde 18d versehen wird, das gleichsinnig mit dem Gewinde 18c des unteren Kolbenstößel-Abschnittes 18a ist, jedoch eine etwas geringere Steigung hat.

Der Kolbenstößel 18 durchgreift in Figur 6 mit seinem zweiten Kolbenstößel-Abschnitt 18b ein Langloch 17, das sich in einem Schieber 14 befindet. Der Schieber 14 ist in einer Ausnehmung 15 des ersten Gehäuseteils 1b verschiebbar angeordnet und kann von außen nach innen gedrückt werden. Der linke Teil des Langloches ist halbkreisförmig und bildet eine Gewindehälfte 17b, deren Gewinde zu dem Gewinde 18d des oberen Kolbenstößel-Abschnittes 18b passend ist. Eine Feder 16 drückt den Schieber 14 nach rechts in die Position, in der die beiden Gewinde ineinandereingreifen.

Wenn der Drehknopf 9 in der durch den gekrümmten Pfeil angedeuteten Richtung gedreht wird, so versucht sich der Kolbenstößel 18 aus dem Kolbenelement 4 heraus nach oben zu drehen. Da das Gewinde 17b im linken Teil des Langloches 17 des Schiebers 14 jedoch eine geringere Steigung hat als das Gewindeloch in dem Kolbenelement 4, wirkt das Gewinde 17b des Langloches 17 auf die Verschiebebewegung des Kolbenstößels 18 bremsend, mit der Folge, dass das Kolbenelement 4 ein kleines Stück nach unten bewegt wird und das negative Tauchvolumen des sich nach oben verschiebenden Kolbenstößels 18 ausgleicht. Die beiden Bewegungen überlagern sich, mit dem Ergebnis, dass sich der Kolbenstößel 18 gegenüber dem Behälter 2 nach oben bewegt und dass sich gleichzeitig das Kolbenelement 4 gegenüber dem Behälter 2 ein - wenn auch sehr kleines - Stück nach unten bewegt, also in Richtung auf das untere Ende des Behälters 2.

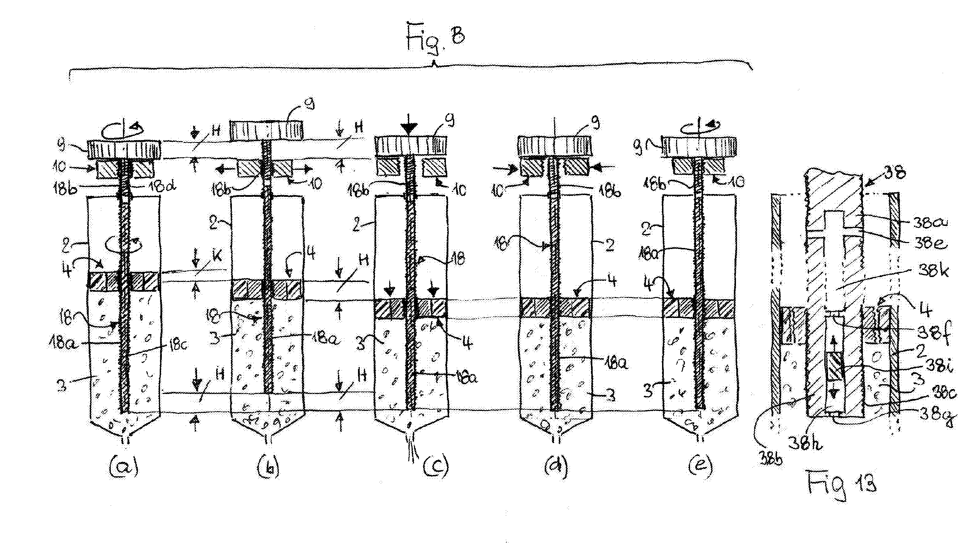

Wenn sich nun der Drehknopf 9 durch Drehen ein der gewünschten Dosierung entsprechendes Stück über der Oberseite des zweiten Gehäuseteiles 1b erhoben hat, so ist die Wendelfeder 20 entsprechend komprimiert worden. Wird daraufhin der Schieber 10 entgegen der Kraft der Feder 16 nach links gedrückt, so gelangt der obere Kolbenstößel-Abschnitt 18b aus dem Wirkungsbereich des Gewindeabschnittes 17b des Langloches 17 mit der Folge, dass die Feder 20 den gesamten Kolbenstößel mit beiden Kolbenstößel-Abschnitten 18a und 18b sowie dem Drehknopf 9 schlagartig nach unten zu bewegen versucht und dabei das Kolbenelement 4 mitnimmt, wodurch der Injektionshub ausgeführt wird, und zwar ohne manuellen Druck auf den Drehknopf 9, wie dies noch in Figur 3(b) und auch in den Figuren 8 (a) - 8(e) angedeutet ist.

Figur 7 zeigt eine zweite Ausführungsform des Getriebemechanismus 10 als Alternative zu Figur 6. Der von außen zu betätigende Schieber 14 trägt hier zwei nach oben ragende Nockenstifte 23, 24, die auf schräge Kanten des hier aus zwei Teilen 21a, 21b bestehenden Ausgleichselementes 21 einwirken und diese bei Druck auf den Schieber 14 entgegen der Federkraft von zwei Schaumgummi-Polstern 22a, 22b nach außen zu drücken vermag. Die beiden Teile 21a, 21b des Ausgleichselementes 21 sind seitlich in Gehäusenuten 25, 26 geführt und haben zwei korrespondierende Gewindehälften, die im zusammengedrückten Zustand der teile 21a, 21b ein Gewindeloch bilden, in dem der obere Kolbenstößel-Abschnitt 18b geführt ist. Wenn die beiden Teile 21a, 21b des Ausgleichselementes 21 auseinander gedrückt werden, so erfolgt eine Entkopplung der Gewinde. Ansonsten ist alles analog zu der ersten Ausführungsform des Getriebemechanismus 10 in Figur 6.

In Figur 8 sind die Phase der Hubvorbereitung und der Hubausführung in vereinfachter Darstellung des Insulin-Pens gemäß dem zweiten Erfindungsvorschlag dargestellt.

Figur 8(a) zeigt das Kolbenelement 4 in einer mittleren Position. Wenn nun der Drehknopf 9 in der durch den gekrümmten Pfeil angedeuteten Richtung gedreht wird, so schraubt sich der Kolbenstößel 18 mit dem Drehknopf 9 nach oben. Gleichzeitig wird das Kolbenelement 4 zum Ausgleich des negativen Tauchvolumens um ein kleines Stück nach unten bewegt. In Figur 7(b) ist die Position des Kolbenelementes 4 und des Drehknopfes 9 nach der Hubvorbereitung dargestellt. Man erkennt, dass der Drehknopf 9 um die Distanz H höher und dass das Kolbenelement 4 um die Distanz K tiefer ist als in Figur 8(a).

Der nächste Schritt ist in Figur 8(b) gezeigt. Hier wird die Ausgleichsvorrichtung 10 geöffnet, sodass sie den Kolbenstößel 18 für eine Vertikalbewegung nach unten frei gibt.

In Figur 8(c) ist gezeigt, wie auf den Drehknopf 9 von oben ein Druck ausgeübt wird und wie der Kolbenstößel und der Drehknopf sich unter Mitnahme des Kolbenelementes 4 nach unten bewegen. Die Bewegung des Kolbenstößels nach unten ist durch die beiden Pfeile oberhalb des Kolbenstößels 4 angedeutet.

Im nächsten Schritt in Figur 8(d) wird das Ausgleichselement 10 wieder geschlossen. Dies ist durch die beiden horizontalen Teile neben dem Ausgleichselement 10 angedeutet. Damit ist der Kolbenstößel 18 für jede weitere Vertikalbewegung ohne Drehung gesperrt.

Der nächste Schritt in Figur 8(e) entspricht wieder der Figur 8(a).

Der Steigerungsunterschied der beiden Gewinde der Kolbenstößel-Abschnitte 18a und 18b lässt sich in einfacher Weise berechnen. Dazu folgendes Beispiel:

S : Steigung des Gewindes 18c des unteren Kolbensstößel-Abschnittes 18a

s : Steigung des Gewindes 18d des oberen Kolbenstößel-Abschnittes 18b

$F = \pi \cdot D^2 / 4$ : Querschnittsfläche des Kolbenelementes 4 (ohne Kolbenstößel 18)

$f = \pi \cdot d^2 / 4$ : Querschnittsfläche des unteren Kolbenstößel-Abschnittes 18a

D : Durchmesser des Kolbenelementes 4

d : Durchmesser des unteren Kolbenstößel-Abschnittes 18a

v : bei einer Hubvorbereitung mit einer Umdrehung des Drehknopfes 9 von dem Kolbenstößel-Abschnitt 18a frei gegebenes Tauchvolumen

V: das durch eine Gegenbewegung des Kolbenelementes 4 eingenommene Ausgleichsvolumen

M3 : metrisches Gewinde mit Durchmesser = 3 mm und Steigung = 0,5 mm

M2,5 : metrisches Gewinde mit Durchmesser = 2,5 mm und Steigung = 0,45 mm

Es soll davon ausgegangen werden, dass das Gewinde des ersten Gewindeteiles 18c das metrische Gewinde M3 ist. Ferner sei D = 10 mm und d = 3 mm. Dann sind:

$$v = f \cdot s \qquad \text{und} \qquad V = F \cdot (S - s)$$

Für den Volumenausgleich muss v = V sein. Dann gilt:

$$F \cdot s = F \cdot (S - s)$$

Daraus kann s wie folgt bestimmt werden:

$$s = S \cdot F / (F + f) \quad \text{oder} \quad s = [S \cdot \pi/4 \cdot (D^2 - d^2)] / \pi/4 \cdot D^2$$

woraus sich die endgültige Formel ergibt:

$$s = S \cdot [1 - (d/D)^2]$$

Mit den oben angegeben Werten ergibt sich für s = 0,5 · [1 - 0,09] · S = 0,445 · S. Diese Gewindesteigung lässt sich in einfacher Weise dadurch realisieren, dass der Durchmesser des 0beren Kobenstößel-Abschnittes 18b auf 2,5 mm reduziert und mit dem genormten metrischen Gewinde M2,5 versehen wird. Es bedarf also keines großen Aufwandes, um den Getriebemechanismus 10 zu realisieren.

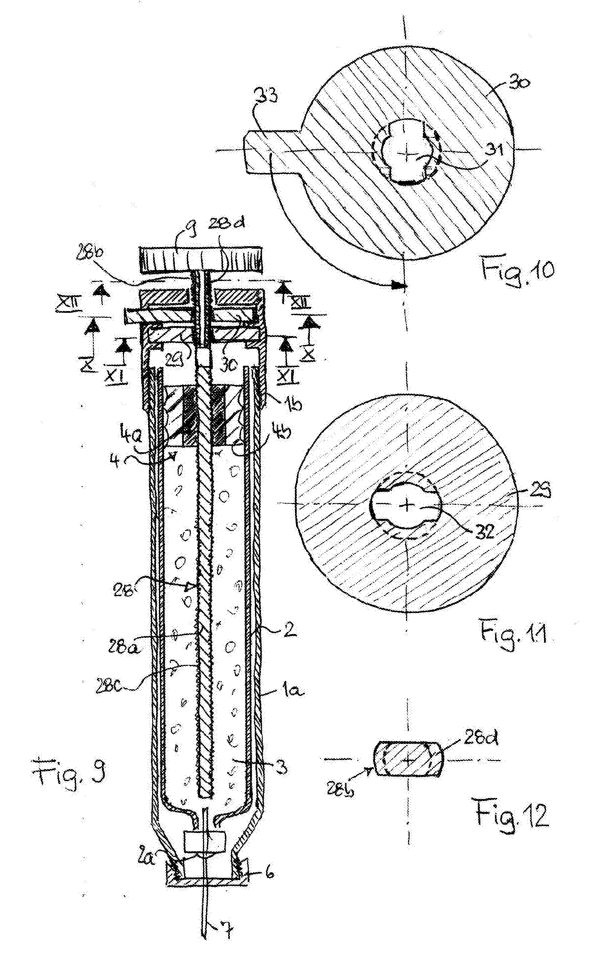

In den Figuren 9 - 12 ist ein schematisierter Pen mit einer dritten Ausführungsform für den Getriebemechanismus zur Realisierung der Ausgleichsmittel gezeigt. Abweichend von den Ausführungsformen gemäß den Figuren 3 - 8 ist der Querschnitt des oberen Kolbenstößel-Abschnittes 28b nicht kreisrund und über den ganzen Umfang mit Gewinde belegt, sondern er ist an zwei gegenüberliegenden Seiten abgeflacht, derart, dass das Gewinde dort entfernt ist. An den zwei anderen gegenüberliegenden Seiten ist das Gewinde 38d dagegen noch vorhanden. Man erkennt das an der Querschnittsdarstellung des Kolbenstößel-Abschnittes 18b in Figur 12.

Im oberen Gehäuseteil 1b ist ein erstes Mutterelement 29 dreh - und verschiebefest verankert, dessen Querschnitt in Figur 11 gezeigt ist. Das Mutterelement enthält eine schlüssellochartige Bohrung 32, die an zwei gegenüberliegenden Randbereichen mit einem Teilgewinde versehen ist, das zu dem Teilgewinde 28d des oberen Kolbenstößel-Abschnittes 28b passend ist. An zwei anderen gegenüberliegenden Stellen ist das Loch 32 gewindefrei. Oberhalb des ersten Mutterteiles 29 ist in dem oberen Gehäuseteil 1b ferner ein zweites Mutterelement 30 angeordnet, das mittels eines über den Gehäuserand hinaus ragenden Hebelansatzes 33 relativ zu dem ersten Mutterelement 29 um die Kolbenstößel-Achse verdrehbar ist. Auch das zweite Mutterelement 30 enthält eine schlüssellochartige Bohrung 31, die gleich wie die Bohrung 32 in dem ersten Mutterteil 29 ist. Im Profil stimmen die beiden Bohrungen 31 und 32 mit dem Querschnitt des oberen Kolbenstößel-Abschnittes 28b überein. Durch Verdrehen des zweiten Mutterelementes 30 gegenüber dem ersten Mutterelement 29 können die beiden Bohrungen 31 und 32 zur Deckung oder außer Deckung gebracht werden. Wenn sie außer Deckung sind, so wirken sie wie ein Gewinde. Wenn sie in Deckung gebracht werden, so kann der obere Kolbenstößel-Abschnitt 28b ohne nennenswerten Widerstand zur Ausführung eines Injektionshubes durch die beiden Bohrungen 31 und 32 hindurch geschoben werden.

Das Schlüssellochprofil muss nicht zwingend so gestaltet sein, wie in den Figuren 10 - 12. Es kann auch drei, vier oder mehr Abflachungen und dazwischen befindliche Teilgewinde haben. An Stelle der Abflachungen können auch nach innen gerichtete Nuten vorgesehen werden.

In Figur 13 ist eine andere Möglichkeit zur Realisierung der Ausgleichsmittel gezeigt, um das frei gegebene Tauchvolumen des Kolbenstößels bei dessen Schraubbewegung nach oben auszugleichen. Man erkennt, dass sich in dem Kolbenstößel 38 eine konzentrische Bohrung 38k befindet, die bis nach oben in den Innenbereich führt, in dem der Kolbenstößel 38 sicher aus dem Behälter 2 zwei herausragt. An dieser Stelle sind in dem Kolbenstößel 38 seitliche Bohrungen 38e vorgesehen, die mit der zentralen Bohrung 38k in Verbindung stehen. Die zentrale Bohrung 38k weist im unteren Teil des Kolbenstößels 38 zwei Verengungen 38f und 38e auf, die einen Ausgleichsraum 38h begrenzen. In diesem Ausgleichsraum 38h ist ein Ausgleichskolben 38i verschiebbar angeordnet. Der untere Teil des Ausgleichsraumes 38h steht demnach mit dem Insulin 3 in dem Behälter 2 in Verbindung, während der obere Teil des Ausgleichsraumes 38h über den zentralen Kanal 38k und die seitlichen Bohrungen 38e mit der Außenatmosphäre in Verbindung steht. Der obere Teil der zentralen Bohrung 38k sowie die radialen Bohrungen 38e bilden demnach Belüftungskanäle. Wenn nun durch das Hochdrehen des Kolbenstößels 38 ein negatives Tauchvolumen entsteht, so wird dieses dadurch ausgeglichen, dass sich - so sei zunächst angenommen - der oben in dem Ausgleichsraum 38h an der oberen Verengung 38f befindliche Ausgleichskolben 38i von dem Insulin 3 nach unten gezogen wird bis er ggf. auf die untere Verengung 38g trifft. Wenn über den Kolbenstößel 38 auf das Kolbenelement 4 wieder ein Druck ausgeübt wird, so treibt das dann ebenfalls unter Druck stehende Insulin 3 den Ausgleichskolben 38i wieder nach oben bis er ggf. auf die Verengung 38f trifft. Die Länge des Ausgleichsraumes 38h muss so bemessen sein, dass sie dem größtmöglichen Hub bei der Hubvorbereitung entspricht.

Der Ausgleichsraum muss sich nicht zwingend in dem Kolbenstößel befinden, sondern er kann beispielsweise auch am Kolben 4 vorgesehen werden.

**Patentansprüche**

1. Injektionsgerät, insbesondere Insulin-Pen, mit
   einem einen Zylinderraum bildenden länglichen Behälter (2) für das Injektionsmittel (3),
   einem in dem Zylinderraum längsverschiebbaren Kolbenelement (4), und
   einem zur Verschiebung des Kolbenelements (4) dienenden Kolbenstößel (8), wobei der Zylinderraum mit einer Injektionskanüle (7) verbindbar ist,
   **dadurch gekennzeichnet,**
   **dass** der Kolbenstößel (8) aus mindestens drei teleskopisch aus- und einfahrbaren Stößelteilabschnitten (8a, 8b, 8c) besteht, die im eingefahrenen Zustand kürzer als der Gesamtverschiebungsweg des Kolbenelementes (4) in dem Behälter (2) ist. (Figur 2 und Figur 4)

2. Injektionsgerät nach Anspruch 1,
   **dadurch gekennzeichnet,**
   **dass** die drei Stößelabschnitte (8a, 8b, 8c) konzentrisch ineinander angeordnet sind und etwa die gleiche Länge haben. (Figuren 2 und 4)

3. Injektionsgerät nach Anspruch 1 oder 2,
   **dadurch gekennzeichnet,**
   **dass** die Stößelabschnitte (8a, 8b, 8c) durch Innen- und/oder Außenschraubgewinde miteinander verbunden sind. (Figuren 2 und 4)

4. Injektionsgerät nach Anspruch 3,
   **dadurch gekennzeichnet,**
   **dass** eine Dosierungsvorrichtung mit Drehmitteln (9) vorgesehen ist, mittels welchen die Stößelteilabschnitte (8a, 8b, 8c) durch gegenseitiges Verdrehen ein- oder ausfahrbar sind. (Figuren 2 und 4)

5. Injektionsgerät nach Anspruch 3 oder 4,
   **dadurch gekennzeichnet,**
   **dass** bei den drei Stößelteilabschnitten (8a, 8b, 8c) der innerste (8a) Außengewinde hat, der mittlere (8b) eine Hülse mit Innen- und Außengewinde ist, und der äußerste (8c) eine Hülse mit Innengewinde ist. (Figuren 2 und 4)

6. Injektionsgerät nach Anspruch 5,
   **dadurch gekennzeichnet,**
   **dass** das Gewinde aller drei Stößelteilabschnitte (8a, 8b, 8c) gleichsinnig ist, und die gleiche Steigung hat. (Figuren 2 und 4)

7. Injektionsgerät, insbesondere Insulin-Pen, mit
   einem einen Zylinderraum bildenden länglichen Behälter (2) für das Injektionsmittel (3),
   einem in dem Zylinderraum längsverschiebbaren Kolbenelement (4), und
   einem zur Verschiebung des Kolbenelements dienenden Kolbenstößel (18; 28; 38), wobei der Zylinderraum mit einer Injektionskanüle (7) verbindbar ist,
   **dadurch gekennzeichnet,**
   **dass** der Kolbenstößel (18; 28; 38) das Kolbenelement (4) durchgreift und in den das Injektionsmittel (3) enthaltenden Teil des Zylinderraums hineintaucht, und dass das Kolbenelement (4) relativ gegenüber dem Kolbenstößel (18; 28; 38) verschiebbar ist. (Figuren 3 und 5-13)

8. Injektionsgerät nach Anspruch 7,

**dadurch gekennzeichnet,**
**dass** der Kolbenstößel (18; 28; 38) mit einem sich über einen Teil seiner Länge erstreckenden ersten Gewindeteil (18c; 28b; 38b) versehen ist, und dass das Kolbenelement (4) ein Gewindeloch aufweist, dessen Gewinde zu dem Gewinde (18c; 28c; 38c) des ersten Gewindeteils (18a; 28a; 38a)) des Kolbenstößels (18; 28; 38) passt. (Figuren 3 und 5 - 13)

9. Injektionsgerät nach Anspruch 7 oder 8,
**dadurch gekennzeichnet,**
**dass** Ausgleichsmittel vorgesehen sind, die - wenn der Kolbenstößel (18; 28; 38) gegenüber dem Kolbenelement (4) zurückbewegt wird - das frei gewordene Tauchvolumen des Kolbenstößels (4) ausgleicht. (Figuren 5-13)

10. Injektionsgerät nach Anspruch 9,
**dadurch gekennzeichnet,**
**dass** die Ausgleichsmittel von einem Getriebemechanismus (10) gebildet sind, der - wenn der Kolbenstößel (18) gegenüber dem Kolbenelement (4) zurückbewegt wird - das Kolbenelement (4) zum Ausgleich des von dem Kolbenstößel (18) freigegeben Tauchvolumens vorwärts bewegt. (Figuren 3, 5-8)

11. Injektionsgerät nach Anspruch 10,
**dadurch gekennzeichnet,**
**dass** der Kolbenstößel (18) einen zweiten Gewindeteil (18b) aufweist, dessen Gewinde zu dem Gewinde eines Gewindeloches in einem Ausgleichselement (14; 21) passt, welches in dem Injektionsgerät in Längsrichtung des Kolbenstößels (18) nicht verschiebbar ist, dass die Gewinde (18c; 18d) der beiden Gewindeteile (18a, 18b) des Kolbenstößels (18) gleichsinnig sind, und dass die Steigung des Gewindes (18d) des zweiten Gewindeteils (18b) geringer ist, als die Steigung des Gewindes (18c) des ersten Gewindeteils (18b). (Figuren 3, 5-8)

12. Injektionsgerät nach Anspruch 11,
**dadurch gekennzeichnet,**
**dass** das Ausgleichselement (14; 21) mit dem Gewinde seines Gewindeloches wahlweise in Kopplungseingriff mit dem Gewinde (18d) des zweiten Gewindeteiles (18b) des Kolbenstößels (18) gebracht bzw. von diesem entkoppelt werden kann. (Figuren 5 - 8)

13. Injektionsgerät nach Anspruch 12,
**dadurch gekennzeichnet,**
**dass** das Ausgleichselement (21) aus mindestens zwei Teilen (21a, 21b) besteht, die zur Entkopplung quer zur Kolbenstößel-Längsachse auseinander bewegt und zur Einkopplung wieder aufeinander zu bewegt werden können. (Figur 7)

14. Injektionsgerät nach Anspruch 12,
**dadurch gekennzeichnet,**
**dass** das Ausgleichselement (10) ein Schieber (14) mit einem Langloch (17) ist, wobei das Langloch (17) aus einem eine Gewindehälfte (17b) bildenden Lochteil und einem gewindefreien Lochteil (17a) besteht. (Figur 6)

15. Injektionsgerät nach Anspruch 12,
**dadurch gekennzeichnet,**
**dass** das Ausgleichselement von zwei in Kolbenstößel-Längsrichtung hintereinander angeordneten Mutterelementen (29, 30) gebildet ist, von denen jedes ein schlüssellochartig geformtes Gewindeloch (31, 32) mit Teilgewinde aufweist, dass die beiden Mutterelemente (29, 309 relativ zueinander verdrehbar sind, derart, dass die Schlüssellochprofile der Gewindelöcher (31, 32) wahlweise zur oder außer Deckung gebracht werden können, und dass der zweite Gewindeteil (28b) des Kolbenstößels (28) - im Querschnitt gesehen - ebenfalls das Schlüssellochprofil mit Teilgewinde aufweist, derart, dass der Kolbenstößel (28) - wenn die Schlüssellochprofile der Gewindelöcher (31, 32) in den beiden Mutterelementen (29, 30) in Deckung sind - von dem Teilgewinde der beiden Gewindelöcher (31, 32) entkoppelt ist und zur Ausführung eines Injektionshubes ungehindert durch die Gewindelöcher (31, 32) in Kolbenstößellängsrichtung hindurch schiebbar ist. (Figuren 9 - 12)

16. Injektionsgerät nach Anspruch 9,
**dadurch gekennzeichnet,**
**dass** die Ausgleichsmittel von einem volumenvariablen Ausgleichsraum (38h) gebildet sind, der mit dem Zylinderraum verbunden ist. (Figur 13)

**17.** Injektionsgerät nach Anspruch 16,
**dadurch gekennzeichnet,**
**dass** der Ausgleichsraum (38h) einen verschiebbaren Ausgleichskolben (38i) enthält, dass der eine auf der einen Seite des Ausgleichskolbens (38i) liegende Teil des Ausgleichsraumes (38h) mit der Außenatmosphäre verbunden ist, und dass der andere auf der anderen Seite des Ausgleichskolbens (38i) liegende Teil des Ausgleichraumes (38h) mit dem das Injektionsmittel (3) enthaltenden Abschnitt des Zylinderraumes verbunden ist. (Figur 13)

**18.** Injektionsgerät nach Anspruch 17,
**dadurch gekennzeichnet,**
**dass** sich der Ausgleichsraum (38h) in dem in das Injektionsmittel eintauchenden Endabschnitt der Kolbenstößel (38) befindet und über einen zentralen Belüftungskanal (38k) in dem Kolbenstößel (38) mit der Außenatmosphäre verbunden ist. (Figur 13)

Fig. 1
Stand der Technik

Fig. 2

Fig. 3

Fig. 4

Fig.5

Fig.7

Fig.6

Fig. 8

Fig. 13

Fig. 9

Fig. 10

Fig. 11

Fig. 12

**Europäisches Patentamt**

# EUROPÄISCHER RECHERCHENBERICHT

Nummer der Anmeldung

EP 07 10 8190

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 97/00091 A (BERNEY JEAN CLAUDE [CH])<br>3. Januar 1997 (1997-01-03)<br>* Abbildungen 1-3 *<br>* Seite 4, Absatz 3 - Seite 9, Absatz 1 *<br>----- | 1-6 | INV.<br>A61M5/315 |
| X | WO 00/15280 A (NJC INNOVATIONS [CH];<br>BERNEY JEAN CLAUDE [CH])<br>23. März 2000 (2000-03-23)<br>* Abbildung 7 *<br>* Seite 13, Zeile 21 - Seite 14, Zeile 12 *<br>* Anspruch 1 *<br>* Seite 6, Zeile 1 - Seite 9, Zeile 25 *<br>----- | 1-6 | |
| X | WO 94/15660 A (BERNEY JEAN CLAUDE [CH])<br>21. Juli 1994 (1994-07-21)<br>* Abbildungen 1,2 *<br>* Seite 4, Absatz 2 - Seite 10, Absatz 1 *<br>----- | 1-6 | |
| X | WO 03/062672 A (NOVO NORDISK AS [DK])<br>31. Juli 2003 (2003-07-31)<br>* Abbildungen 1-9 *<br>* Seite 6, Zeile 11 - Seite 15, Zeile 34 *<br>----- | 1-4 | |
| X | EP 0 462 508 A (ISHIKAWA TOICHI [JP]; KATO HATSUJO KAISHA LTD [JP])<br>27. Dezember 1991 (1991-12-27)<br>* Abbildungen 1-6 *<br>* Spalte 2, Zeile 38 - Spalte 5, Zeile 49 *<br>----- | 1 | RECHERCHIERTE SACHGEBIETE (IPC)<br>A61M |
| X | US 3 082 914 A (WILBUR GILL)<br>26. März 1963 (1963-03-26) | 7-10 | |
| A | * Abbildungen 1-4 *<br>* Spalte 1, Zeile 6 - Spalte 2, Zeile 64 *<br>----- | 11-18 | |
| | -/-- | | |

Der vorliegende Recherchenbericht wurde für alle Patentansprüche erstellt

| Recherchenort | Abschlußdatum der Recherche | Prüfer |
|---|---|---|
| München | 27. Juli 2007 | Reinbold, Sylvie |

EPO FORM 1503 03.82 (P04C03)

**Nummer der Anmeldung**

EP 07 10 8190

**Europäisches**
**Patentamt**

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 01/52923 A (SCIMED LIFE SYSTEMS INC [US]) 26. Juli 2001 (2001-07-26) * Abbildungen 1-6 * * Seite 4, Zeile 9 - Seite 10, Zeile 20 * ----- | 7 | |
| A | US 2 349 726 A (HOLLER GEORGE J) 23. Mai 1944 (1944-05-23) * Abbildungen 1-6 * * Seite 1, Zeile 1 - Seite 2, Spalte 1, Zeile 48 * ----- | 7-18 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 27. Juli 2007 | Reinbold, Sylvie |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**Europäisches**
**Patentamt**

Nummer der Anmeldung

EP 07 10 8190

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☒ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

**Europäisches**
**Patentamt**

**MANGELNDE EINHEITLICHKEIT**
**DER ERFINDUNG**
**ERGÄNZUNGSBLATT B**

Nummer der Anmeldung

EP 07 10 8190

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-6

    Diese Ansprüche offenbaren ein Injektionsgerät:
    - mit einem Behälter
    - mit einem Kolbenelement
    - mit einem Kolbenstössel
    - dass der Kolbenstössel aus mindestens drei teleskopisch aus- und einfahrbaren Stösselteilabschnitten besteht (technischer Effekt: ein kompakteres Injektionsgerät zur Verfügung stellen )
    ---

2. Ansprüche: 7-18

    Diese Ansprüche offenbaren ein Injektionsgerät:
    - mit einem Behälter
    - mit einem Kolbenelement
    - mit einem Kolbenstössel
    - dass der Kolbenstössel das Kolbenelement durchgreift und in den das Injektionsmittel enthaltenden Teil des Zylinderraums hineintaucht, und dass das Kolbenelement relativ gegenüber dem Kolbenstössel verschiebbar ist. (technischer Effekt: eine Alternative von einem kompakteren Injektionsgerät zur Verfügung stellen )
    ---

**ANHANG ZUM EUROPÄISCHEN RECHERCHENBERICHT
ÜBER DIE EUROPÄISCHE PATENTANMELDUNG NR.**

EP 07 10 8190

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-07-2007

| Im Recherchenbericht angeführtes Patentdokument | | Datum der Veröffentlichung | Mitglied(er) der Patentfamilie | | Datum der Veröffentlichung |
|---|---|---|---|---|---|
| WO 9700091 | A | 03-01-1997 | EP | 0851774 A1 | 08-07-1998 |
| WO 0015280 | A | 23-03-2000 | KEINE | | |
| WO 9415660 | A | 21-07-1994 | CH | 685461 A3 | 31-07-1995 |
| WO 03062672 | A | 31-07-2003 | AT | 310185 T | 15-12-2005 |
| | | | DE | 60302335 D1 | 22-12-2005 |
| | | | DE | 60302335 T2 | 10-08-2006 |
| | | | EP | 1472477 A1 | 03-11-2004 |
| | | | JP | 2005515383 T | 26-05-2005 |
| EP 0462508 | A | 27-12-1991 | JP | 4051966 A | 20-02-1992 |
| | | | US | 5178609 A | 12-01-1993 |
| US 3082914 | A | 26-03-1963 | KEINE | | |
| WO 0152923 | A | 26-07-2001 | AU | 783866 B2 | 15-12-2005 |
| | | | AU | 3647201 A | 31-07-2001 |
| | | | CA | 2397700 A1 | 26-07-2001 |
| | | | EP | 1248656 A1 | 16-10-2002 |
| | | | JP | 2003520108 T | 02-07-2003 |
| | | | US | 2003018298 A1 | 23-01-2003 |
| | | | US | 2004147873 A1 | 29-07-2004 |
| US 2349726 | A | 23-05-1944 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0245312 B1
- EP 0450905 B1
- EP 0496141 B1
- EP 0829268 A2
- EP 1644061 B1
- DE 3716340 C2
- DE 19730999 C1
- US 4865591 A
- WO 9938554 A
- EP 0110687 B1
- EP 0721358 B1
- EP 0909194 B1